# EUROPÄISCHE PATENTSCHRIFT

(11) **EP 1 872 772 B1**
(45) Veröffentlichungstag und Bekanntmachung des Hinweises auf die Patenterteilung: **05.08.2015**
(21) Anmeldenummer: 07016471.0
(22) Anmeldetag: 12.08.2004
(51) Int. Cl.: A61K 8/49, A61K 31/4188, A61P 17/00, A61K 8/67, A61Q 19/02, A61K 31/375

(54) **Verwendung von Biotin oder einem Biotinderivat zur Aufhellung der Haut und/oder zur Ausglättung von farblichen Hautunebenheiten**
Use of biotin or a biotin derivative for lightening skin and/or for smoothening skin color irregularities
Utilisation de biotine ou d'un dérivé de biotine pour éclaircir la peau et/ou pour atténuer les irrégularités de couleur de peau

(30) Priorität: 26.08.2003 EP 03018730
(43) Veröffentlichungstag der Anmeldung: 02.01.2008
(62) Teilanmeldung aus: 04764050.3
(73) Patentinhaber: DSM IP Assets B.V., 6411 TE Heerlen (NL)
(72) Erfinder: Jermann, Roland, 4242 Laufen (CH); Luther, Helmut, 79639 Grenzach-Wyhlen (DE)
(74) Vertreter: Best, Michael

(56) Entgegenhaltungen:
- EP-A- 1 449 514
- WO-A1-97/25972
- US-A- 4 584 191
- US-A- 5 519 039
- US-A- 5 710 177
- US-B1- 6 436 414
- DATABASE WPI Week 2004 Derwent Publications Ltd., London, GB; AN 2004-191087 XP002457676 "Internal medicament containing tranexamic acid and e.g. licorice, biotin to treat pigment deposition such as Riehl's disease, lichen planus, vitiligo, freckles or age-related liver spots." & WO 2004/006908 A (TAKADA H,TAKADA T, TAKEMURA T) 22. Januar 2004 (2004-01-22)

## Beschreibung

Die vorliegende Erfindung betrifft die nicht-therapeutische, kosmetische Verwendung von Biotin oder einem Biotinderivat alleine, bevorzugt aber zusammen mit Vitamin C oder einem Vitamin C-Derivat, zur Ausglättung von farblichen Hautunebenheiten und/oder zur Aufhellung der natürlichen Hautfarbe.

Hautaltersflecken sind Dunkelflecken auf der Haut, die mit der Alterung der Haut entstehen. Sie sind eine Folge von verschiedenen Alterungsprozessen, die durch Lichteinstrahlung beschleunigt werden. Die Haut erscheint so farblich inhomogen.

Die Hautbräunung ist eine natürliche Schutzfunktion der Haut, welche bei verschiedenen ethnischen Gruppen unterschiedlich stark ausgeprägt ist. In vielen Kulturkreisen gilt eine helle Hautfarbe als attraktiv, so daß ein Bedürfnis nach Aufhellung der natürlichen Hautfarbe besteht.

Mittel zur Hautaufhellung sind bekannt, wie z.B. Hydrochinon, Kojisäure, Arbutin, Vitamin C sowie diverse pflanzliche Extrakte. Ein Problem bei vielen Mitteln besteht jedoch darin, daß neben einer Aufhellung der Haut oder einer Beseitigung von Altersflecken Nebenwirkungen wie z.B. Hautirritationen auftreten können. Pflanzliche Extrakte, die weniger hautirritativ sind, zeigen meistens eine nicht ausreichende Wirksamkeit.

Es besteht Bedarf nach weiteren Mitteln, insbesondere kosmetischen Mitteln, die gut hautverträglich sind und dennoch wirksam zur Hautaufhellung und zur Ausglättung von farblichen Hautunebenheiten sind. Die Mittel sollten eine mindestens ebenso gute, bevorzugt eine bessere Wirksamkeit aufweisen wie die bekannten Mittel zur Hautaufhellung.

Biotin ist ein bekannter Wirkstoff, der in zahlreichen kosmetischen Formulierungen und Arzneimitteln vorhanden ist. Bei Biotin handelt es sich um die Verbindung 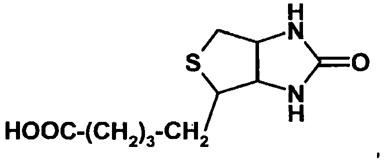 die in acht verschiedenen stereoisomeren Formen vorkommen kann. Insbesondere wird unter Biotin das D-(+)-Biotin verstanden, also die Verbindung (3aS,4S,6aR)-2-Oxohexahydrothieno[3,4-d]-imidazol-4-valeriansäure der Formel

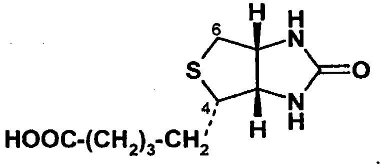

Eine Wirksamkeit von Biotin zur Hautaufhellung ist bislang nicht bekannt.

Erfindungsgemäß wurde überraschend gefunden, daß Biotin einen hautaufhellenden Effekt aufweist und damit zur Ausglättung von farblichen Hautunebenheiten sowie zur Aufhellung der natürlichen Hautfarbe verwendet werden kann.

Die Erfindung stellt damit die Verwendung von Biotin zur Herstellung eines Mittels zur Aufhellung der natürlichen Hautfarbe und zur Ausglättung von farblichen Hautunebenheiten zur Verfügung.

Die Bezeichnung "Biotin" bezieht sich erfindungsgemäß auf die acht Stereoisomeren der Formel 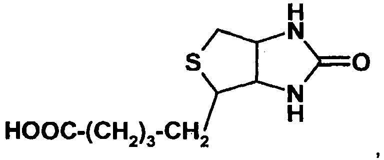 entweder in stereochemisch reiner Form oder als beliebige Mischung von zwei oder mehreren der Stereoisomeren. Besonders bevorzugt ist erfindungsgemäß das D-(+)-Biotin der Formel

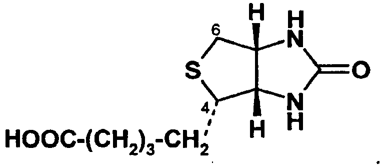

Biotinderivate sind dem Fachmann bekannt. Hierunter werden erfindungsgemäß Biotinester der Formeln I und II verstanden, die sich wie folgt aus Biotin herleiten: 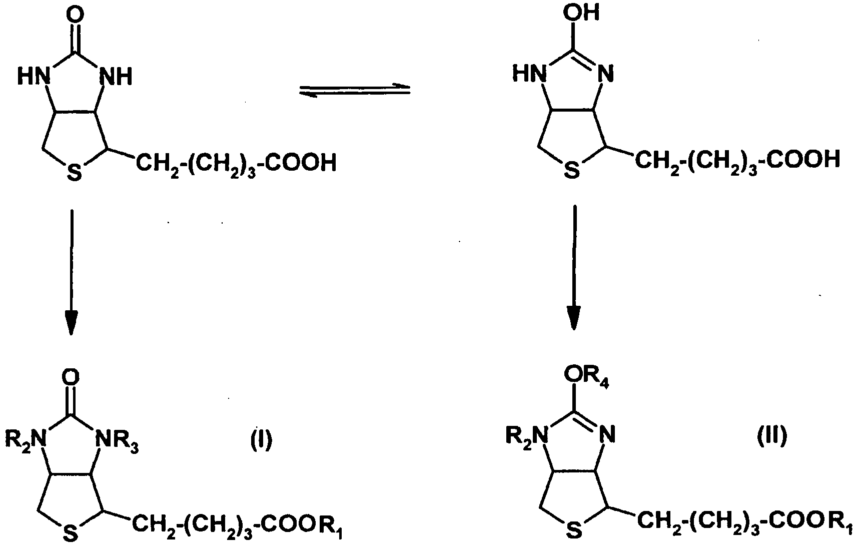 R₁ = H, C₁-C₂₀-Alkyl, C₅-C₇-Cycloalkyl, Aryl
R₂ und R₃ = unabhängig voneinander H, C₁-C₅-Alkoxycarbonyl
R₄ = H, C₁-C₂₀-Alkyl, C₁-C₅-Alkoxycarbonyl.

Ein C₁-C₂₀-Alkylrest ist bevorzugt ein C₁-C₁₀-Alkylrest, stärker bevorzugt ein C₁-C₆-Alkylrest, wie eine Methyl-, Ethyl-, n-Propyl-, iso-Propyl-, n-Butyl-, tert.-Butyl- oder iso-Butylgruppe.

Ein C₅-C₇-Cycloalkylrest ist bevorzugt eine Cyclohexylgruppe.

Ein Arylrest ist bevorzugt ein C₅-C₁₀-Arylrest, insbesondere eine Phenylgruppe.

Ein C₁-C₅-Alkoxycarbonylrest ist bevorzugt ein C₁-C₃-Alkoxycarbonylrest.

Bevorzugt ist der Rest R₁ ein Wasserstoffatom oder ein C₁-C₆-Alkylrest.

Bevorzugt ist zumindest einer der Reste R₂ oder R₃ ein Wasserstoffatom, stärker bevorzugt sind beide Rest R₂ und R₃ Wasserstoffatome. Der Rest R₄ ist bevorzugt ein Wasserstoffatom oder ein C₁-C₆-Alkylrest, stärker bevorzugt ein Wasserstoffatom.

Besonders bevorzugt sind die Reste R₂, R₃ und R₄ alle Wasserstoffatome, und der Rest R₁ ist ein C₁-C₆-Alkylrest, wie vorstehend definiert.

Auch von den Biotinderivaten sind erfindungsgemäß alle stereoisomeren Formen und Salze entweder einzeln oder in beliebigen Gemischen umfaßt.

Erfindungsgemäß können Biotin und die Derivate davon einzeln verwendet werden, es ist aber auch möglich, ein Gemisch aus Biotin und einem oder mehreren seiner Derivate einzusetzen, beispielsweise Biotin im Gemisch mit einem oder mehreren Biotinestern, wie vorstehend definiert. Ebenso können verschiedene Biotinderivate im Gemisch miteinander eingesetzt werden.

Die Herstellung der Biotinderivate ist dem Fachmann bekannt, und hier können übliche Standardmethoden der organischen Chemie eingesetzt werden, z.B. die Veresterung von Biotin mit dem gewünschten Alkohol wie Methanol oder Ethanol unter Wasserabspaltung.

Das Biotin und die Biotinderivate können ebenfalls in der Salz-Form eingesetzt werden. Geeignete Biotinsalze sind nicht besonders eingeschränkt, und hier können Salze mit Alkalien, Erdalkalien und andern geeigneten Metallen, aber auch mit Ammonium und organischen Basen, insbesondere Natrium-, Kalium-, Kalzium- und Magnesiumsalze, genannt werden. Aufgrund der Stickstoffatome kann das Biotin und insbesondere auch die Biotinderivate auch in Form eines Säureadditionssalzes nach Umsetzung mit einer geeigneten Säure, wie einer anorganischen oder organischen Säure, insbesondere einer Mineralsäure, z.B. mit HCl, vorliegen. Das Hydrochloridsalz ist besonders bevorzugt. Die Herstellung der Salze erfolgt auf an sich bekannte Art und Weise z.B. durch Umsetzung des Biotins oder des Derivats davon mit der entsprechenden Base (z.B. NaOH oder KOH) oder der entsprechenden Säure (z.B. HCl).

Soweit im Rahmen dieser Beschreibung von einem "Mittel" gesprochen wird, ohne daß eine genauere Spezifizierung erfolgt, wird hierunter ein kosmetisches Mittel verstanden. Zur Abgrenzung zwischen kosmetischen Mitteln und Arzneimitteln kann z.B. auf Römpp, Chemielexikon, 10te Auflage und die darin zitierte Literatur verwiesen werden. Erfindungsgemäß bevorzugt wird Biotin zur Herstellung eines kosmetischen Mittels eingesetzt, bei dem das Biotin zusammen mit kosmetisch verträglichen Zusatzstoffen formuliert wird. Sofern im Rahmen dieser Anmeldung nichts anderes dargelegt wird, sind genannte Zusatzstoffe kosmetisch verträgliche Zusatzstoffe.

Erfindungsgemäße bevorzugt handelt es sich bei den Mitteln, zu denen Biotin formuliert wird, um topische Mittel, wie z.B. flüssige oder feste Öl-in-Wasser-Emulsionen, Wasser-in-Öl-Emulsionen, Mehrfachemulsionen, Mikroemulsionen, PIT-Emulsionen, Bickering-Emulsionen, Hydrogele, alkoholische Gele, Lipogele, ein- oder mehrphasige Lösungen, Schäume, Salben, Pflaster, Suspensionen, Puder, Cremes, Cleanser, Seifen und andere übliche Mittel, die auch z.B. über Stifte, Masken oder als Sprays verabreicht werden können.

Die topischen Mittel umfassen einen oder mehrere kosmetisch verträgliche bzw. pharmazeutisch verträgliche Träger und/oder Zusatz- bzw. Hilfsstoffe, wie sie üblicherweise in derartigen Zubereitungen verwendet werden. Hier können beispielsweise Fette, Öle, Wachse, Silikone, Emulgatoren, Alkohole, Polyole, Verdickungsmittel, anfeuchtende und/oder feuchthaltende Substanzen, Tenside, Weichmacher, Schaumbremsen, anionische, kationische, nichtionische oder amphotere Polymere, Alkanisierungs- oder Azidifizierungsmittel, Enthärter, Adsorbentien, Lichtschutzmittel, Elektrolyte, Sequestrierungsmittel, Wasser, organische Lösungsmittel, Konservierungsmittel, Bakterizide, Antioxidantien, Vitamine, Duftstoffe, Aromen, Süßungsmittel, Farbstoffe und Pigmente genannt werden.

Die erfindungsgemäßen topischen Formulierungen enthalten bevorzugt einen oder mehrere übliche Fettstoffe, z.B. pflanzliche Öle, flüssige Paraffinöle, Isoparaffinöle, synthetische Kohlenwasserstoffe, Di-n-alkylester, Fettsäuren, Fettalkohole, Esteröle, Hydroxycarbonsäurealkylester, Dicarbonsäureester, Diolester, symmetrische, unsymmetrische oder cyclische Ester oder Kohlensäureester mit Fettalkoholen, Mono-, Di- und Trifettsäureester mit Glycerin, Wachse und Siliconverbindungen.

Die Fettstoffe sind in der Regel in einer Menge von 0,1 bis 50 Gew.-%, bevorzugt von 0,1 bis 20 Gew.-%, insbesondere von 0,1 bis 15 Gew.-% in dem topischen Mittel vorhanden (jeweils bezogen auf das gesamte Mittel).

Die topischen Mittel können eine oder mehrere oberflächenaktive Substanzen als Emulgatoren oder Dispergiermittel enthalten. Geeignete Beispiele derartiger Emulgatoren bzw. Dispergiermittel sind bekannt.

Die Emulgatoren können in den topischen Mitteln beispielsweise in Anteilen von 0,1 bis 25 Gew.-%, stärker bevorzugt von 0,5 bis 15 Gew.-%, bezogen auf das gesamte Mittel, vorhanden sein.

Die topischen Mittel können ebenfalls übliche Lichtschutzmittel, beispielsweise übliche UV-A- und/oder UV-B-Filter, enthalten. Eine Zusammenstellung üblicher UV-A- und UV-B-Filter, die auch in den erfindungsgemäßen Mitteln eingesetzt werden können, findet sich beispielsweise in der EP-A 1 081 140. Erfindungsgemäß können selbstverständlich auch die in dieser Druckschrift erstmals offenbarten neuen Sonnenschutzfilter in den erfindungsgemäßen Mitteln eingesetzt werden.

Geeignete organische, mineralische oder modifizierte mineralische Lichtschutzfilter sind auch in der WO 01/64177 angegeben, auf die insoweit ebenfalls Bezug genommen wird.

Falls gewünscht, können die erfindungsgemäßen Mittel ebenfalls Proteinhydrolysate oder Derivate davon sowie geeignete Mono-, Oligo- oder Polysaccharide oder deren Derivate enthalten, wie sie z.B. in der WO 01/64177 offenbart sind. Weitere geeignete Hilfs- und Zusatzstoffe wie Vitamine, Provitamine und Vitaminvorstufen, Alantopin, Bisabolol, Antioxidantien, Ceramide und Pseudoceramide, Triterpene, monomere Catechine, Verdickungsmittel, Pflanzenglycoside, strukturgebende Mittel (Strukturanten), Dimethylisosorbid, Lösungsmittel, Quell- und Penetrationshilfsstoffe, Parfümöle, Pigmente und Farbstoffe zum Anfärben des Mittels, Substanzen zur Einstellung des pH-Werts, Komplexbildner, Trübungsmittel, Perlglanzmittel, Treibmittel, filmbildende, emulsionsstabilisierende, verdickende oder adhäsive Polymere, insbesondere kationische, anionische sowie nicht-ionische Polymere, sind ebenfalls in der WO 01/64177 offenbart, auf die insoweit Bezug genommen wird.

Die Mittel sind bevorzugt derart formuliert, daß sie zur topischen Verabreichung geeignet sind. Die topische Verabreichung erfolgt bevorzugt mindestens einmal pro Tag, z.B. zwei- oder dreimal am Tag. Die Behandlungsdauer beträgt in der Regel mindestens zwei Tage bis die gewünschte Wirkung erzielt wurde. Die Behandlungsdauer kann auch mehrere Wochen oder Monate betragen.

Die Menge des aufzutragenden Mittels richtet sich nach der Konzentration des Wirkstoffs in dem Mittel sowie dem gewünschten kosmetischen Erfolg. Eine geeignete Menge für die Anwendung hängt von der Beschaffenheit der Haut, der zu behandelnden Person sowie von der Schwere und Art der zu behandelnden Hautverfärbung und anderen Faktoren ab, die einem behandelnden Kosmetiker bekannt sind. Beispielsweise kann die Auftragung derart erfolgen, daß eine Creme auf die Haut aufgetragen wird. Eine Creme wird üblicherweise in einer Menge von 2 mg Creme / cm² Haut aufgetragen. Die aufgetragene Menge ist aber nicht kritisch, und sollte sich mit einer bestimmten aufgetragenen Wirkstoffmenge kein Behandlungserfolg einstellen, kann die aufgetragene Menge ohne weiteres erhöht werden, beispielsweise, indem höher konzentrierte topische Formulierungen verwendet werden.

Erfindungsgemäß kann der Wirkstoff als solcher oder auch in enkapsulierter Form, zum Beispiel in liposomaler Form, formuliert werden. Liposome werden vorteilhafterweise mit Lecithinen ohne oder mit Zusatz von Sterolen oder Phytosterolen gebildet. Die Enkapsulierung des Wirkstoffs kann alleine oder zusammen mit anderen Wirkstoffen erfolgen.

In dem erfindungsgemäßen Mittel ist das Biotin in einer Menge von 0,0001 Gew.-% bis etwa 50 Gew.-% bezogen auf das Gesamtgewicht des Mittels vorhanden. Stärker bevorzugt ist Biotin in einer Menge von 0,01 Gew.-% bis etwa 20 Gew.-%, noch stärker bevorzugt in einer Menge von etwa 0,01 Gew.-% bis etwa 1 Gew.-%, insbesondere in einer Menge von etwa 0,1 Gew.-% bezogen auf das Gesamtgewicht des Mittels vorhanden.

Zur Art und Herstellung der topischen Mittel sowie zur Offenbarung beispielhafter Zusatzstoffe kann auf die einschlägige Literatur verwiesen werden, z.B. auf NOWAK G.A., Die kosmetischen Präparate, - Band 2, Die kosmetischen Präparate - Rezeptur, Rohstoffe, wissenschaftliche Grundlagen (Verlag für chem. Industrie H. Ziolkowsky KG, Augsburg).

Es ist ebenfalls möglich, Biotin als orales Mittel zu formulieren, beispielsweise in Form von Pillen, Tabletten, Kapseln, die z.B. ein Granulat oder Pellets enthalten, als flüssige orale Formulierungen oder als Zusatzstoff zu Nahrungsmitteln, wie es dem Fachmann prinzipiell bekannt ist. Geeignete Verfahren und Zusatzstoffe, mit denen die oral zu verabreichenden Mittel erfindungsgemäß hergestellt werden können, sind z.B. in dem Standardwerk "Remington: The Science and Practice of Pharmacy", Lippincott, Williams und Wilking (Herausgeber) 2000 offenbart, auf das insoweit Bezug genommen wird.

Als übliche Zusatzstoffe für orale Mittel, insbesondere für Tabletten, können übliche Exzipienten wie mikrokristalline Cellulose, Natriumcitrat, Calciumcarbonat, Dinatrium- oder Dikaliumphosphat, Natrium- oder Kaliumphosphat, Glycin, Sprengmittel wie Stärke oder Alginsäure, Bindemittel wie Polyvinylpyrrolidon, Saccharose, Gelatine und Gummi arabicum, Gleitmittel wie Magnesiumstearat, Natriumlaurylsulfat oder Talkum für die Tablettierung verwendet werden. Werden die Mittel in Gelatinekapseln gefüllt, sind übliche Hilfsstoffe für die Herstellung von Granulat Laktose oder Milchzucker sowie Polyethylenglykole mit hohem Molekulargewicht. Weitere Hilfs- und Zusatzstoffe sowie geeignete Hilfs- und Zusatzstoffe für andere orale Formulierungen, insbesondere auch für die Formulierung als Nahrungsmittelzusatzstoffe, sind dem Fachmann bekannt, und es kann auf die einschlägige Literatur verwiesen werden, z.B. auf Grundzüge der Lebensmitteltechnik, Horst-Dieter Tscheuschner (Hrsg.) 2., neubearb. Aufl. Hamburg: Behr's 1996.

Im Falle eines oralen Mittels beträgt der Gehalt an Wirkstoff (d.h. an Biotin bzw. Biotinderivat) in dem Mittel üblicherweise 1 % bis 90%, bevorzugt 10% bis 80%, z.B. 50% oder mehr. Die Verabreichung erfolgt derart, daß die gewünschte Wirkung eintritt und hängt von dem Zustand des Patienten, der Art und Schwere der zu behandelnden Hautverfärbung, etc. ab und kann von einem Fachmann leicht bestimmt werden. Eine übliche tägliche Dosierung liegt in einem Bereich von 0.1 µg/Tag bis 50 mg/Tag, z.B. 20 µg/Tag bis 2 mg/Tag.

Erfindungsgemäß wurde weiterhin überraschend gefunden, daß Biotin neben der eigenen Wirksamkeit zur Hautaufhellung eine unerwartet hohe hautaufhellende Wirkung zeigt, wenn es zusammen mit Vitamin C oder einem Vitamin C-Derivat verabreicht wird.

Vitamin C-Derivate sind bekannt, und hierunter werden erfindungsgemäß alle Verbindungen verstanden, die in vivo oder in vitro Vitamin C freisetzen, sowie Solvate, Hydrate und Salze davon. Als Beispiele für Vitamin C-Derivate können Glukoside der Ascorbinsäure und Phosphate der Ascorbinsäure und insbesondere Magnesiumascorbylphosphat, Natriumascorbylphosphat, Calciumascorbylphosphat, Kaliumascorbylphosphat und gemischte Salze, wie z.B. Natriummagnesiumascorbylphosphat oder Natriumcalciumascorbylphosphat genannt werden. Insbesondere die Phosphate liegen häufig als Hydrate vor, wobei die Dihydratform die gebräuchlichste ist. Besonders bevorzugt wird Biotin erfindungsgemäß zusammen mit Natriumascorbylphosphat eingesetzt und am stärksten bevorzugt in der Form des Dihydrats, wie es beispielsweise unter der Produktbezeichnung STAY-C 50 von der Firma Roche Vitamins AG erhältlich ist.

Es war bekannt, daß Vitamin C eine hautaufhellende Wirksamkeit zeigt, es war jedoch nicht bekannt, daß eine Kombination von Biotin und Vitamin C bzw. einem Vitamin C-Derivat eine hautaufhellende Wirksamkeit zeigt, die weitaus stärker ausgeprägt ist, als die hautaufhellende Wirksamkeit des Vitamin C allein.

Erfindungsgemäß kann das Vitamin C bzw. das Derivat davon in die gleiche Formulierung eingearbeitet werden, in der auch das Biotin vorliegt. Vitamin C bzw. das Derivat davon wird bevorzugt in einer Menge von 0,001 Gew.-% bis etwa 50 Gew.-% bezogen auf das Gesamtgewicht des Mittels in einem topischen Mittel eingesetzt. Stärker bevorzugt ist Vitamin C bzw. das Derivat davon in einem topischen Mittel in einer Menge von 0,01 Gew.-% bis etwa 20 Gew.-%, noch stärker bevorzugt in einer Menge von etwa 0,1 Gew.-% bis etwa 15 Gew.-%, z.B. 1 bis etwa 5 Gew.-%, wie etwa 3 Gew.-%, bezogen auf das Gesamtgewicht des Mittels vorhanden. Bezüglich der Menge an Vitamin C bzw. des Derivats davon in einem oralen Mittel kann auf die vorstehenden Ausführungen zu Biotin verwiesen werden, die ebenfalls für die Menge und Dosierung des Vitamin C bzw. des Derivats davon gilt.

Erfindungsgemäß wird unter dem Ausdruck "Mittel" auch eine Ausführungsform verstanden, in der das Mittel in zwei separaten Teilen vorliegt, wobei ein Teil den Wirkstoff Biotin und der andere Teil das Vitamin C oder das Derivat davon enthält. Bei den beiden separaten Teilen des Mittels kann es sich jeweils um topisch aufzutragende Mittel handeln oder jeweils um oral zu verabreichende Mittel, es ist aber auch möglich, daß ein separater Teil des Mittels topisch aufzutragen ist und der andere Teil des Mittels oral zu verabreichen ist, so daß in dem erfindungsgemäßen Mittel z.B. ein separater Teil den Wirkstoff Biotin enthält und topisch zu verabreichen ist, während der andere separate Teil den Wirkstoff Vitamin C oder ein Derivat davon enthält und oral zu verabreichen ist oder wobei der separate Teil des Mittels, der den Wirkstoff Biotin enthält, oral zu verabreichen ist und der separate Teil des Mittels, der den Wirkstoff Vitamin C bzw. das Derivat davon enthält, topisch zu verabreichen ist.

Für die Herstellung, die Inhaltsstoffe, die Wirkstoffgehalte und die Dosierungen der separaten Teile des Mittels kann auf die vorstehenden Ausführungen zu den topischen und oralen Formulierungen mit Biotin verwiesen werden, die entsprechend auch für die erfindungsgemäße Ausführungsform gelten, in der das Mittel in zwei separaten Teilen vorliegt, die jeweils einen Wirkstoff enthalten. Die Menge an Vitamin C bzw. des Derivats davon in dem anderen separaten Teil ist entsprechend der Ausführungsform, in der das Mittel nicht in Form von zwei separaten Teilen vorliegt, sondern beide Wirkstoffe im Gemisch vorliegen, so daß auf die vorstehenden Ausführungen verwiesen werden kann.

Sofern das Mittel sowohl Biotin als auch Vitamin C oder ein Derivat davon enthält, ist das Gewichtsverhältnis des Vitamin C zu Biotin vorzugsweise 500:1 bis 1:500, stärker bevorzugt 100:1 bis 1:100 und insbesondere 30:1 bis 1:30. Es ist weiterhin bevorzugt, daß die Menge an Vitamin C bzw. des Derivats davon in dem Mittel höher ist als die Menge an Biotin. Die vorstehenden Angaben gelten sowohl für die Ausführungsformen, in denen Biotin und Vitamin C bzw. ein Derivat davon im Gemisch miteinander vorliegen als auch für Ausführungsformen, bei denen das Mittel aus zwei separaten Teilen besteht, wobei der eine Teil den Wirkstoff Biotin und der andere Teil den Wirkstoff Vitamin C bzw. ein Derivat davon enthält.

Soweit die hier genannten Wirkstoffe als Hydrate oder Solvate vorliegen können, sind auch die Hydrate und Solvate von der vorliegenden Erfindung umfaßt.

Bevorzugt wird erfindungsgemäß ein Mittel, das beide Wirkstoffe im Gemisch miteinander enthält, besonders bevorzugt ist ein topisch zu verabreichendes Mittel.

Die folgenden Beispiele erläutern die Erfindung.

### 1. Formulierungsbeispiel

Aus folgenden Bestandteilen wurde auf an sich bekannte Art und Weise eine Creme hergestellt.

| | **Inhaltsstoffe** | **INCI Benennung** | **% w/w** |
|---|---|---|---|
| **A)** | Brij 721 | Steareth 21 | 4,00 |
| | Brij 72 | Steareth 2 | 2,00 |
| | Lanette O | Cetearyl Alcohol | 2,00 |
| | Glyceryl Myristate | Glyceryl Myristate | 3,00 |
| | Oleic Acid | Oleic Acid | 6,00 |
| | Tegosoft M | Isopropyl Myristate | 3,00 |
| | Estol 1517 | Isopropyl Palmitate | 3,00 |
| | Transcutol CG | Ethoxydiglycol | 5,00 |
| | Phenonip | Phenoxyethanol & Methylparaben & Ethylparaben & Propylparaben & Butylparaben | 0,80 |
| | Dow Corning 200, 350 cs | Dimethicone | 0,50 |
| | BHT | Butylated Hydroxytoluene | 0,05 |
| | | | |
| **B)** | Deionized water | Aqua | Ad 100 |
| | Propylene Glycol | Propylene Glycol | 5,00 |
| | Edeta BD | Disodium EDTA | 0,10 |
| | Keltrol T | Xanthan Gum | 0,20 |
| | Carbopol ETD 2001 | Carbomer | 0,30 |
| | | | |
| **C)** | TEA 99% | Triethanolamine | qs pH 7 |
| | Biotin | Biotin | 0,10 |
| | Deionized water | Aqua | 10,00 |
| | | | |
| **D)** | Deionized water | Aqua | 6,00 |
| | STAY-C 50 | Sodium Ascorbyl Phosphate | 3,00 |

Teile A) und B) wurden getrennt voneinander unter Rühren jeweils auf 75°C erwärmt. Sobald die Teile A) und B) homogen waren, wurde Teil B) dem Teil A) unter Rühren zugefügt. Es wurde mit 11000 UpM. 30 Sekunden homogenisiert. Teil C) wurde gemischt auf 65°C vorerwärmt und zu dem homogenisierten Gemisch aus A) und B) zugegeben. Das Gemisch aus A), B) und C) wurde auf 40°C abgekühlt, und Teil D) wurde zugesetzt. Es wurde unter Rühren auf Umgebungstemperatur (25°C) abgekühlt.

Die entstandene Creme hatte einen pH-Wert von 7,0 und zeigte eine Viskosität (Brookfield RVT, 25°C, Spindel 5, 10 UpM) von etwa 20.000 cP.

Auf entsprechende Art und Weise wurde neben einer erfindungsgemäßen Creme ein Placebo hergestellt, bei dem weder Natriumascorbylphosphat noch Biotin vorhanden waren, sowie eine Creme mit ausschließlich 0,1 % Biotin und eine Creme mit ausschließlich 3% Natriumascorbylphosphat.

### 2. Testbeispiel

39 weibliche Probanden wurden in drei Gruppen zu je 13 Personen aufgeteilt. Die Probanden applizierten während drei Monaten zweimal täglich je eine Testformulierung auf die linke und eine zweite auf die rechte Gesichtshälfte sowie auf den linken und den rechten Handrücken. Die Testformulierungen waren codiert und entsprachen einer Placeboformulierung und einer Formulierung mit der gewünschten Testsubstanz. Die drei Gruppen testeten dabei die vorstehend hergestellten Cremes mit 3% Natriumascorbylphosphat (STAY-C 50), 0,1% Biotin und mit einem Gemisch aus 3% Natriumascorbylphosphat und 0,1% Biotin.

Zur Messung der Aufhellung der Altersflecken wurde ein Chromometer CR 300 verwendet. Die erhaltenen Werte wurden als ITA°-Wert angegeben. ITA° beschreibt den Pigmentierungsgrad der Haut bzw. der Altersflecken. Die nachtstehend wiedergegebenen Werte entsprechen den Differenzen des ITA° gegenüber dem Basiswert vor Beginn der Studie. Je höher der Wert, desto stärker die Aufhellung der Haut. Die ITA°-Wert wurden nach 29, 57 und 85 Tagen, das heißt nach etwa einem Monat, nach etwa zwei Monaten und nach etwa drei Monaten bestimmt. Die Ergebnisse sind in folgender Tabelle aufgeführt.

| Produkt | ITA° | | | ITA°-p-Werte | | |
|---|---|---|---|---|---|---|
| | Tag 29 | Tag 57 | Tag 85 | Tag 29 | Tag 57 | Tag 85 |
| Placebo | 1,53 | 7,67 | 9,29 | 0,381 | 0,953 | 0,857 |
| 3% NAP | 4,46 | 7,53 | 8,80 | | | |
| Placebo | 2,75 | 5,93 | 8,07 | 0,156 | 0,480 | 0,217 |
| 0,1% Biotin | 5,57 | 7,55 | 11,16 | | | |
| Placebo | 3,32 | 6,89 | 9,70 | 0,055 | 0,006 | 0,045 |
| 3% NAP + 0,1 % Biotin | 7,16 | 11,65 | 13,42 | | | |

Die Studie wurde während der Wintermonate durchgeführt, und in dieser Zeit hellt sich die Haut auf natürliche Art auf. Damit ist zu erklären, daß auch die Placeboformulierungen eine leichte Hautaufhellung zeigen. Der Hautaufhellungseffekt für die Placeboformulierungen ist aber nur gering.

Überraschenderweise zeigt Biotin bereits in einer Konzentration von 0,1% einen Hautaufhellungseffekt, der nach einem und drei Monaten höher ist und nach zwei Monaten etwa genauso hoch ist wie der des bekannten Hautaufhellungsmittels Vitamin C. Besonders überraschend ist die sehr hohe hautaufhellende Wirksamkeit eines Gemisches aus 3% Natriumascorbylphosphat und 0,1 % Biotin.

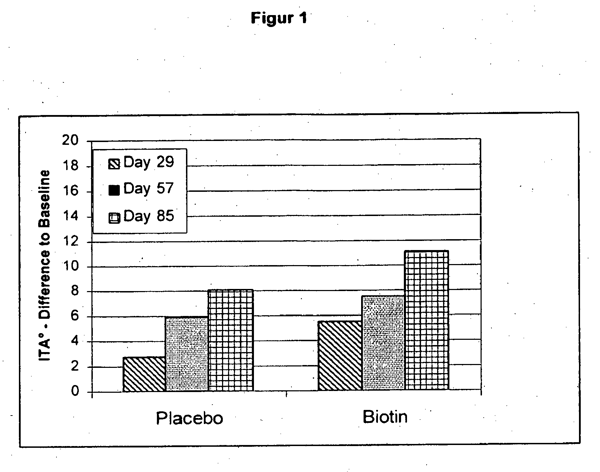

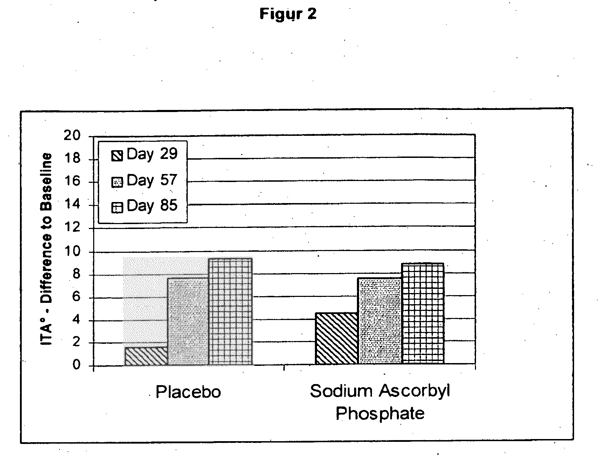

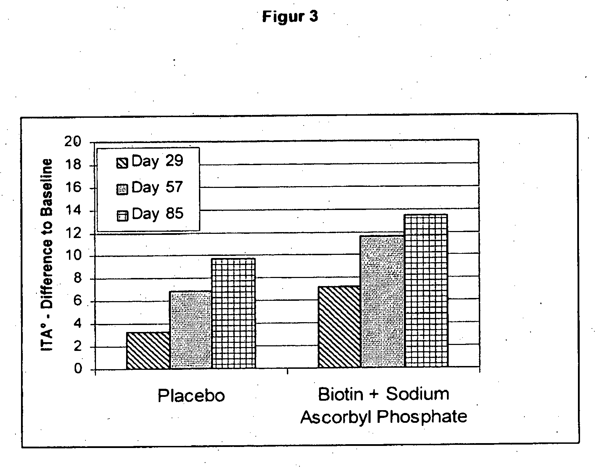

Die Ergebnisse der Studie sind in den Figuren 1 bis 3 dargestellt.

## Patentansprüche

1. Nicht-therapeutische kosmetische Verwendung von Biotin, eines Biotinderivats oder eines Salzes davon zur Aufhellung der Haut und/oder zur Ausglättung von farblichen Hautunebenheiten, wobei es sich bei dem Biotinderivat um eine Verbindung der Formel I oder der Formel II handelt 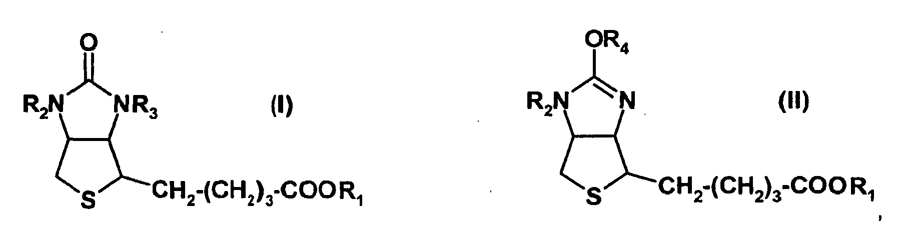 wobei
R₁ = H, C₁-C₂₀-Alkyl, C₅-C₇-Cycloalkyl, Aryl
R₂ und R₃ = unabhängig voneinander H, C₁-C₅-Alkoxycarbonyl und
R₄ = H, C₁-C₂₀-Alkyl, C₁-C₅-Alkoxycarbonyl ist.

2. Verwendung nach Anspruch 1, wobei es sich um eine topische Verwendung handelt.

3. Verwendung nach Anspruch 1 oder 2, wobei es sich beim Biotinsalz um ein Alkalisalz, Erdalkalisalz oder ein Ammoniumsalz oder um ein Hydrochlorid des Biotins oder des Biotinderivats handelt.

4. Verwendung nach einem der Ansprüche 1 bis 3, wobei das Biotin, das Biotinderivat oder das Salz davon Bestandteil eines Mittels ist und darin in einer Konzentration von 0,001 bis 50 Gew.-%, bezogen auf das Gewicht des Mittels, vorhanden ist.

5. Verwendung nach Anspruch 4, wobei das Biotin, das Biotinderivat oder das Salz davon in einer Konzentration von 0,001 bis 1 Gew.-% bezogen auf das Gewicht des Mittels, vorhanden ist.

## Claims

1. Non-therapeutic cosmetic use of biotin, a biotin derivative or a salt thereof for lightening the skin and/or for smoothening skin color irregularities, wherein the biotin derivative is a compound of formula I or formula II 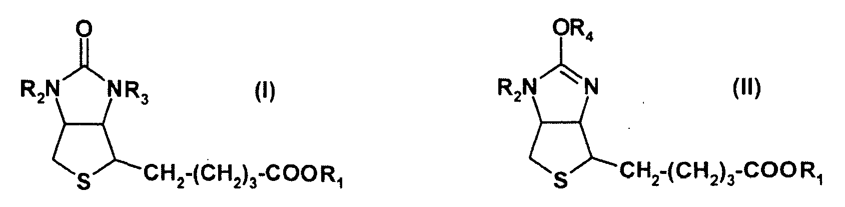 wherein
R₁ = H, C₁-C₂₀-alkyl, C₅-C₇-cycloalkyl, aryl
R₂ and R₃ = each independently H or C₁-C₅-alkoxycarbonyl and
R₄ = H, C₁-C₂₀-alkyl, C₁-C₅-alkoxycarbonyl.

2. Use according to claim 1, wherein the use is a topical use.

3. Use according to claim 1 or 2, wherein the biotin salt is an alkali salt, an alkaline earth salt or an ammonium salt or a hydrochloride of the biotin or the biotin derivative.

4. Use according to any of claims 1 to 3, wherein the biotin, the biotin derivative or the salt thereof is a component of an agent and is present therein at a concentration of 0.001 to 50% by weight, in relation to the weight of the agent.

5. Use according to claim 4, wherein the biotin, the biotin derivative or the salt thereof is present at a concentration of 0.001 to 1 % by weight, in relation to the weight of the agent.

## Revendications

1. Utilisation cosmétique non thérapeutique de biotine, d'un dérivé de biotine ou d'un sel de celui-ci pour éclaircir la peau et/ou pour atténuer des irrégularités de couleur de la peau, le dérivé de biotine étant un composé de formule I ou de formule II 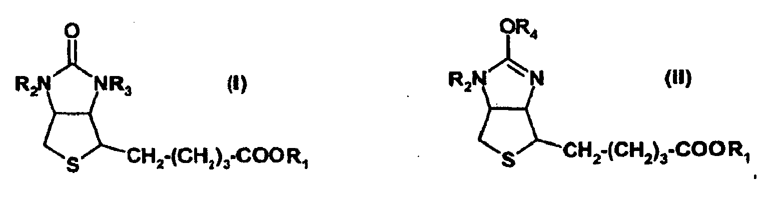 dans lesquelles
R₁ = H, alkyle en C₁-C₂₀, cycloalkyle en C₅-C₇, aryle
R₂ et R₃ = indépendamment l'un de l'autre H, alkoxycarbonyle en C₁-C₅ et
R₄ = H, alkyle en C₁-C₂₀, alkoxycarbonyle en C₁-C₅.

2. Utilisation selon la revendication 1, celle-ci étant une utilisation topique.

3. Utilisation selon la revendication 1 ou 2, dans laquelle le sel de biotine est un sel alcalin, un sel alcalino-terreux ou un sel d'ammonium ou étant un hydrochlorure de la biotine ou du dérivé de biotine.

4. Utilisation selon l'une quelconque des revendications 1 à 3, la biotine, dans laquelle le dérivé de biotine ou le sel de celui-ci fait partie d'un produit et y est présent dans une concentration de 0,001 à 50 % en poids par rapport au poids du produit.

5. Utilisation selon la revendication 4, dans laquelle la biotine, le dérivé de biotine ou le sel de celui-ci est présent dans une concentration de 0,001 à 1 % en poids par rapport au poids du produit.
